# FASCICULE DE BREVET EUROPEEN

(11) **EP 0 655 945 B1**
(45) Date de publication et mention de la délivrance du brevet: **01.07.1998**
(21) Numéro de dépôt: 93917852.1
(22) Date de dépôt: 04.08.1993
(51) Int. Cl.: B01J 13/02

(54) **PROCEDE DE FABRICATION DE MICROPARTICULES EN EMULSION PAR MODIFICATION DE LA COMPOSITION CHIMIQUE DE LA PHASE DISPERSEE APRES EMULSIFICATION**
VERFAHREN ZUR HERSTELLUNG VON MIKROPARTIKELN IN EMULSIONEN DURCH MODIFIZIERUNG DER CHEMISCHEN ZUSAMMENSETZUNG DER DISPERSEN PHASE NACH EMULGIERUNG
METHOD FOR FABRICATING MICROPARTICLES IN EMULSION BY MODIFICATION OF THE CHEMICAL COMPOSITION OF THE DISPERSED PHASE AFTER EMULSIFICATION

(30) Priorité: 20.08.1992 FR 9210174
(43) Date de publication de la demande: 07.06.1995
(73) Titulaire: COLETICA, 69007 Lyon (FR)
(72) Inventeur: ORLY, Isabelle, F-69002 Lyon (FR); LEVY, Marie-Christine, F-51100 Reims (FR); PERRIER, Eric, F-38200 Vienne (FR)
(74) Mandataire: Portal, Gérard
(86) Numéro de dépôt international: FR9300791
(87) Numéro de publication internationale: WO9404260

(56) Documents cités:
- EP-A- 0 273 823
- GB-A- 1 135 856
- US-A- 4 187 194
- US-A- 4 217 370
- US-A- 4 497 793

## Description

La présente invention concerne essentiellement un procédé de fabrication de microparticules en émulsion par modification de la composition chimique de la phase dispersée après émulsification. Avantageusement, cette fabrication de microparticules est obtenue par réaction chimique ou physicochimique au sein de la phase dispersée d'une émulsion d'une substance ou d'un mélange de substances, par modification de la composition chimique de la phase dispersée après émulsification par incorporation d'un agent chimique essentiellement insoluble dans le liquide dispersant, pour provoquer ladite réaction.

Il est connu de former des microparticules par réaction chimique, par exemple, par les documents FR-A-2 444 497 Mars, FR-A-2 527 438 CNRS, ou encore BIOETICA (= EP-A-0 381 543) qui décrivent la préparation de microcapsules par réticulation interfaciale d'une solution aqueuse de protéine ou d'un mélange de protéine et de polysaccharide au sein d'une phase hydrophobe à l'aide d'un agent bifonctionnel tel que les dichlorures d'acides.

Le document GB-A-1,135,856 est relatif à un procédé de modification des alginates alkylèneglycol pour la formation d'agents de mise en suspension, d'agents liants et d'adhésifs en particulier pour lier des matériaux en poudre ou granule, la formation de revêtements de surface résistant à l'eau, des gelées élastiques (page 2, ligne 121 à la page 3, ligne 104), ou dans les produits cosmétiques pour utiliser cette capacité liante.

Ce document ne concerne donc en aucune façon le domaine technique de la fabrication de microcapsules ou microparticules contenant dans leur masse un polysaccharide estérifié, par un procédé en émulsion.

Il est également connu de fabriquer des microparticules par modifications physicochimiques à partir de solutions de polymères mais il s'agit de procédés de coacervation dits de coacervation simple ou de coacervation complexe (document EP 0 273 823 A1 MERO ROUSSELOT SATIA, par exemple), selon que l'on fait intervenir respectivement un ou deux types de polymères. Dans ce document, la réaction de coacervation a lieu dans la phase dispersante conduisant ainsi à la formation d'une membrane qui se dépose sur les gouttelettes du liquide hydrophobe dispersé ou sur les particules du solide dispersé. Le procédé décrit dans ce document est, par ailleurs, limité à l'encapsulation d'un liquide hydrophobe ou d'un solide, l'encapsulation d'une phase aqueuse n'étant pas possible.

Le document US-A-4 217 370 RAWLINGS décrit un procédé de piégeage de microgouttelettes lipidiques dispersées dans une phase dispersante à base de substance protéique qui est précipitée par une modification de pH. Ainsi, ce procédé consiste à emprisonner des gouttelettes dans une matrice rigidifiée par précipitation de la phase dispersante.

Le document US-A-4 187 194 WELLMAN décrit un procédé d'encapsulation par évaporation de solvant.

Le document US-A-4 497 593 SIMKIN est relatif à un procédé classique de réticulation interfaciale selon lequel aucune modification chimique n'est réalisée au sein de la phase dispersée.

La présente invention permet de fabriquer des microparticules par une méthode différente des méthodes classiques de réticulation interfaciale ou de coacervation simple et complexe.

La présente invention a pour but de réaliser la fabrication de microparticules, en particulier de microcapsules, par un procédé de fabrication extrêmement simple, peu coûteux, présentant une bonne reproductibilité, donc une bonne fiabilité, permettant de régler la dimension des particules obtenues dans un large domaine de valeurs et surtout donnant la possibilité d'éviter l'utilisation d'agents réticulants bifonctionnels dont la présence résiduelle ou les produits de réaction sont susceptibles de compromettre la biocompatibilité des microparticules ou microcapsules ainsi formées, en le rendant ainsi très compétitif à l'échelle industrielle et de préférence adapté pour un usage cosmétique ou pharmaceutique ou encore alimentaire. L'invention a pour but de modifier la composition chimique ou physicochimique de la phase dispersée pour fabriquer des microparticules, en particulier des microcapsules.

La présente invention permet de résoudre simultanément ces exigences.

Ainsi, la présente invention fournit un procédé de fabrication de microparticules, en particulier de microcapsules, caractérisé en ce que l'on prépare tout d'abord une solution essentiellement homogène d'une substance ou d'un mélange de substances dans un solvant, que l'on réalise une émulsion de la solution dans un liquide dispersant formant une phase continue dans lequel ladite substance ou ledit mélange sont essentiellement insolubles, et formant une phase dispersée puis on déclenche une réaction chimique ou physicochimique dans la phase dispersée par modification de la composition chimique in situ de ladite substance ou dudit mélange dans la phase dispersée, en ajoutant un agent essentiellement non soluble ou non miscible dans ou à la phase continue, dans les conditions d'addition, en provoquant ainsi une modification de l'état physicochimique résultant en une insolubilisation de la substance ou du mélange de substances et en l'individualisation desdites microparticules, en présence du solvant de départ, lesdites microparticules étant ensuite récupérées.

L'objet de la présente invention repose essentiellement sur la possibilité parfaitement nouvelle pour l'homme de l'art d'être capable de modifier la composition chimique de la phase dispersée d'une émulsion après que la dispersion ait été réalisée et ceci par l'intermédiaire d'agents non solubles ou non miscibles dans ou à la phase continue.

Selon une variante de réalisation avantageuse, l'agent essentiellement non soluble ou non miscible dans ou à la phase continue, dans les conditions d'addition, que l'on souhaite incorporer à la phase dispersée est présent à l'état dissous dans un solvant miscible au solvant de la phase dispersée et présentant une affinité pour le liquide dispersant de la phase continue inférieure à l'affinité vis-à-vis de la phase dispersée.

Selon une application particulière, cette opération permet, de façon tout à fait inattendue, de faire diffuser l'agent permettant le déclenchement de la réaction à travers une phase hydrophobe jusque dans les gouttelettes d'une solution aqueuse dispersée, alors qu'une solution aqueuse du même agent est parfaitement inefficace.

Comme substance ou mélange de substances utilisées pour la réalisation de la solution, on choisit de préférence des protéines, des polysaccharides ou des acides nucléiques, des mélanges de protéines, des mélanges de polysaccharides ou des mélanges de protéines et de polysaccharides, éventuellement avec des acides nucléiques.

Comme protéines, on peut citer les protéines purifiées suivantes : alpha-lactalbumine, bêta-lactoglobuline, caséines, ovalbumine, albumines animales, globulines sanguines, hémoglobine, fibrinogène, collagène, atélocollagène, gélatine, kératine, albumines végétales, globulines végétales, gluténines, gliadine ; les extraits protéiques issus du lait, de la soie, des céréales, des légumineuses, des algues, du poisson.

Comme polysaccharides, on peut citer l'agar, l'agarose, l'agaropectine, les carraghénanes, les alginates, les pectines, l'amylose, l'amylopectine, l'amidon, les amidons modifiés, les galactomannanes (guar, caroube), le glucomannane, le konjac, les celluloses modifiées, l'inuline, le xanthane, le dextrane, le curdlane, la gellane, le chitosan, les chondroïtines sulfates, l'acide hyaluronique, le dermatane sulfate, l'héparane sulfate, l'héparine, le kératane sulfate.

Comme acides nucléiques, on envisage l'acide ribonucléique et l'acide désoxyribonucléique.

Comme réaction chimique, on envisage la formation de liaisons covalentes en particulier des estérifications et des amidations ou la formation de liaisons ioniques entre les groupements ionisables d'une substance ou d'un mélange de substances.

Comme réaction physicochimique, on entend essentiellement des réactions telles que des réactions de coacervation, de précipitation, ou d'insolubilisation.

Selon une autre variante de réalisation avantageuse du procédé, celui-ci est caractérisé en ce que l'agent essentiellement non soluble ou non miscible précité dans ou à la phase continue conduit à une séparation de phase au sein de la solution initialement homogène de la phase dispersée, ou à une gélification de ladite solution de la phase dispersée ou à une perte de solubilité de la phase dispersée par condensation ou par polymérisation.

Selon une autre variante de réalisation avantageuse du procédé, l'agent essentiellement non soluble ou non miscible précité dans ou à la phase continue, dans les conditions d'addition, est un non solvant pour la substance ou le mélange de substances solubilisée(s) dans le solvant de la phase dispersée, ou ledit agent conduit à une modification de pH, ou ledit agent comprend au moins un électrolyte, ou ledit agent comprend au moins une molécule susceptible de réagir avec la ou les substances dissoutes dans la phase dispersée de l'émulsion.

Selon encore une autre variante de réalisation avantageuse, le non solvant précité pour la substance ou le mélange de substances solubilisée(s) dans le solvant de la phase dispersée, et essentiellement non miscible à la phase continue dans les conditions d'addition, provoque une insolubilisation de la substance ou du mélange de substances de la phase dispersée, en particulier une gélification ou une coagulation.

Selon encore une autre variante de réalisation particulière, le non solvant précité est choisi parmi un alcool, en particulier un alcool inférieur en C₁-C₆, de préférence l'alcool éthylique, ou une cétone, en particulier une cétone inférieure en C₂-C₆, de préférence l'acétone.

Selon un premier mode de réalisation avantageux, on réalise les étapes sucessives suivantes :
a) on prépare une solution aqueuse d'une substance choisie parmi les acides nucléiques, une protéine ou un polysaccharide ou les divers mélanges de ces substances ;
b) on prévoit un liquide hydrophobe dans lequel la substance ou les substances précitées sont essentiellement insolubles ;
c) on mélange le liquide hydrophobe et la phase aqueuse pour former une émulsion dans laquelle la solution aqueuse est la phase dispersée et le liquide hydrophobe est la phase continue ;
d) on ajoute à l'émulsion le non solvant de la substance solubilisée dans la phase dispersée, dans des proportions telles que ledit non solvant soit essentiellement non miscible à la phase continue en formant ainsi des microparticules par gélification ou coagulation ;
e) on recueille par des moyens de séparation physique, par exemple par filtration, centrifugation ou décantation, les microparticules formées par ladite gélification ou ladite coagulation ;
f) de préférence, on renforce la cohésion des microparticules par une réticulation.

Selon un deuxième mode de réalisation avantageux, on réalise tout d'abord une émulsion d'une solution aqueuse d'une substance ou d'un mélange de substances susceptibles de subir par réaction chimique ou physicochimique une variation d'état physicochimique traduite par une insolubilisation provoquée par une modification de pH, à un pH auquel l'état physicochimique de ladite substance ou dudit mélange de substances est celui d'une solution de viscosité appropriée à la réalisation d'une émulsion ; puis, on fait varier le pH, en ajoutant l'agent non soluble ou non miscible précité, comprenant une substance modifiant le pH dissoute dans un solvant organique miscible à la phase aqueuse, pour déclencher la réaction chimique ou physicochimique et amener la substance ou le mélange de substances dans un état physicochimique correspondant à une insolubilisation de la substance ou du mélange et qui conduit à la formation de microparticules physiquement individualisées.

Comme substance modifiant le pH, on utilise en général un acide, une base, ou un tampon en fonction de la valeur de pH souhaitée.

Selon une caractéristique avantageuse des procédés selon l'invention, la substance utilisée si l'on souhaite alcaliniser la phase dispersée de l'émulsion est une solution de soude ou de potasse dans un alcool tel que le méthanol ou l'éthanol, utilisés purs ou renfermant 5 à 10 % (p/v) d'eau, ou encore un polyol, tel que le glycérol ou un polyéthylèneglycol. Selon une caractéristique préférée, la solution contient entre 0,5 et 10 % de soude dans l'éthanol 95 % (p/v).

Selon une autre caractéristique avantageuse des procédés selon l'invention, la substance utilisée si l'on souhaite acidifier la phase dispersée de l'émulsion est une solution d'un acide monocarboxylique ou polycarboxylique, porteur ou non de fonctions alcool, comme l'acide acétique, l'acide citrique, l'acide lactique, l'acide tartrique, l'acide succinique, l'acide malique, ou d'un acide minéral comme l'acide chlorhydrique, dans un alcool tel que le méthanol ou l'éthanol, purs ou renfermant 5 à 10 % d'eau, ou encore dans un polyol, tel que le glycérol ou un polyéthylèneglycol. Selon une caractéristique préférée, la solution acide est constituée d'éthanol à 95 % contenant entre 1 et 10 % (v/v) d'acide acétique.

Selon un autre aspect, la réaction provoquée in situ par le changement de pH dans les gouttelettes de la phase dispersée est une réaction physicochimique de coacervation.

Dans ce cas, le procédé de fabrication des microparticules est avantageusement le suivant :
a) on prépare une solution aqueuse d'une substance choisie parmi les acides nucléiques, un polysaccharide ou une protéine ou les divers mélanges de ces substances, le pH étant choisi de façon que la substance ou le mélange de substances forme une solution essentiellement homogène, de viscosité compatible avec la réalisation d'une émulsion ;
b) on prévoit un liquide hydrophobe dans lequel la substance ou les substances précitées sont essentiellement insolubles ;
c) on mélange le liquide hydrophobe et la phase aqueuse pour former une émulsion ;
d) on ajoute à l'émulsion soit une solution d'une substance alcaline dans un liquide organique miscible à la phase aqueuse si l'on souhaite alcaliniser la phase aqueuse ou une solution d'une substance acide dans un liquide organique miscible à la phase aqueuse si l'on souhaite acidifier la phase aqueuse ;
e) après une période de temps prédéterminée nécessaire pour réaliser la coacervation de la ou des substances initialement solubilisées, on recueille les microparticules formées par centrifugation ou par filtration ;
f) de préférence, on renforce la cohésion des microparticules par une réticulation. La réticulation est préférée dans la majorité des cas.

Parmi les agents -classiques pour l'homme de l'art- de réticulation des composés polyaminés, polycarboxylés ou polyhydroxylés ou présentant un mélange de ces types de groupements, on citera à titre d'exemple un certain nombre de réactifs qui peuvent être regroupés en deux types.

Le premier type est constitué des agents bifonctionnels qui forment un pontage entre les groupements réactifs de la ou des substances constitutives des microparticules et subsistent dans le produit de réaction. Le formaldéhyde, le glutaraldéhyde, et les di-aldéhydes en général, les dichlorures d'acides, les di-anhydrides d'acides, les di-isocyanates, les di-imidoesters, les bis-chloroformiates, les succinimides font partie de ce type de réactifs.

Le second type de réactifs concerne les agents qui ne forment pas de pontage mais qui activent les groupements carboxyliques en particulier, de façon qu'ils puissent former des liaisons amides avec les groupements aminés ou des liaisons esters avec les fonctions alcools de la substance ou du mélange de substances mis en oeuvre pour la fabrication des microparticules. Les méthodes aux azides telles que décrites dans les documents EP-A-0301977 BIOETICA et WO 90/12055 BIOETICA ou utilisant des carbodiimides, par exemple, sont représentatives de ce type de méthodes.

Dans le cas où l'on souhaite préparer des microparticules de parfaite biocompatibilité, ce qui est absolument indispensable pour une utilisation pharmaceutique, on préférera particulièrment l'utilisation des méthodes du second type qui, permettant la formation de liaisons amides ou esters directement entre les groupements carboxyliques d'une part et aminés ou alcools d'autre part de la ou des substances constitutives des microparticules, conduisent à l'obtention tout à fait remarquable de microparticules constituées uniquement de la ou des substances mises en oeuvre pour la réalisation de l'émulsion, à l'exclusion de tout agent de réticulation.

Selon une variante particulière, la protéine utilisée est du collagène, en effet cette protéine est essentiellement soluble à pH inférieur à 4 et précipite à des pH supérieurs à cette valeur. On fabrique donc une solution de collagène à un pH voisin de 3,5. On réalise une émulsion par dispersion de la solution de collagène dans un liquide hydrophobe. On alcalinise les gouttelettes de la solution de collagène par addition à l'émulsion d'une solution alcoolique alcaline ce qui provoque une précipitation du collagène et ainsi une individualisation de microparticules de collagène que l'on peut récupérer par centrifugation ou par filtration et auquel on peut ensuite faire subir une réticulation selon les méthodes précitées.

Selon une caractéristique avantageuse de l'invention, la concentration de la solution de collagène est comprise entre 0,1 et 2 %, de préférence voisine de 0,5 % (p/v).

Selon une seconde variante particulière la protéine utilisée est un dérivé de collagène, de préférence l'atélocollagène.

Selon une caractéristique avantageuse de l'invention, la concentration de la solution d'atélocollagène est comprise entre 0,3 et 4 % (p/v).

Selon une troisième variante particulière, le polysaccharide utilisé est un glycosaminoglycanne bien connu à l'homme de l'art. Les glycosaminoglycannes sont en particulier décrits dans le document antérieur du déposant EP-A-0318154. Le polysaccharide utilisé peut également être le chitosan. Le chitosan est un polymère polyionique qui n'est soluble en phase aqueuse qu'à des pH inférieurs à 5. Lorsque le pH d'une solution de chitosan est amené à un pH proche de la neutralité, le polymère précipite en formant des fibrilles parfaitement insolubles dans l'eau. On peut tirer profit de cette propriété pour la réalisation de microcapsules selon l'invention. On prépare une solution de chitosan à pH acide, on réalise une émulsion dans un liquide hydrophobe, on modifie le pH de la phase dispersée par ajout, à l'émulsion, d'une base en solution alcoolique. La précipitation in situ permet la formation de microparticules facilement récupérables que l'on peut réticuler ultérieurement de façon chimique si nécessaire.

Selon une caractéristique avantageuse de l'invention, on préfère un chitosan hautement désacétylé et de très haut poids moléculaire, de préférence supérieur à 300 000 daltons. Par "hautement désacétylé" on entend de préférence un taux d'acétylation résiduel inférieur à environ 5 %, mieux 3,5%. De tels chitosans hautement désacétylés sont disponibles dans le commerce.

Selon une caractéristique avantageuse de l'invention, la concentration de la solution de chitosan est comprise entre 0,2 et 10 %, de préférence voisine de 1 % (p/v).

Selon un second aspect de formation de sphères par variation du pH de la phase dispersée d'une émulsion, la formation de microcapsules est réalisée par déclenchement, une fois que l'émulsion est réalisée, d'une réaction chimique entre les groupements réactionnels d'une substance ou de substances mélangées.

La réaction chimique peut être, par exemple, une réaction de transacylation qui se produit à pH alcalin entre un polysaccharide porteur de groupements carboxyliques estérifiés, et soit une substance polyaminée comme par exemple une protéine, soit une substance polyhydroxylée comme par exemple un polysaccharide.

Dans ce cas, le procédé de fabrication des microparticules est avantageusement le suivant :
a) on prépare une solution aqueuse neutre donc non réactive renfermant d'une part un polysaccharide porteur de groupements carboxyliques estérifiés, et d'autre part soit une substance polyaminée comme par exemple une protéine, soit une substance polyhydroxylée comme par exemple un polysaccharide ;
b) on prévoit un liquide hydrophobe dans lequel le polysaccharide estérifié et la substance polyaminée ou polyhydroxylée sont essentiellement insolubles ;
c) on mélange le liquide hydrophobe et la solution aqueuse pour former une émulsion ;
d) on ajoute à l'émulsion une solution d'une substance alcaline dans un liquide organique miscible à la phase aqueuse, ce qui permet l'obtention de conditions physicochimiques favorables au déroulement de la réaction entre la substance polyaminée ou la substance polyhydroxylée et le polysaccharide porteur des groupements carboxyliques estérifiés ;
e) après une période de temps prédéterminée nécessaire pour réaliser une réaction de transacylation, en formant ainsi des microparticules, en particulier des microcapsules, on réalise une neutralisation de l'émulsion, de préférence en ajoutant à l'émulsion une solution d'une substance acide dans un liquide organique miscible à la phase aqueuse ; ce qui neutralise et stabilise les microparticules, en particulier les microcapsules formées.

Selon une variante de réalisation, on forme une émulsion de la solution aqueuse comme phase dispersée dans le liquide hydrophobe formant la phase continue.

Selon un second mode de mise à profit de l'invention, la modification de composition chimique induite in situ après réalisation de l'émulsion, est une modification de la concentration en certains électrolytes.

Un certain nombre de polysaccharides s'associent spécifiquement avec des ions ou des polyions pour former des gels.

En particulier un certain nombre de polysaccharides polyanioniques s'associent spécifiquement avec des cations ou des polycations, plus particulièrement avec des cations monovalents comme le potassium et le sodium ou bivalents comme le calcium, le magnésium, le strontium ou le baryum. Cette association se traduit par la formation de gels plus ou moins cassants en fonction de la nature du polysaccharide, du cation et des concentrations mises en oeuvre.

Selon une variante particulière, on réalisera un mélange de polysaccharides de façon à pouvoir moduler la cohésion du gel formé.

Comme polysaccharide préférentiellement utilisé, on chosit les carraghénanes kappa et iota, les pectines faiblement méthylées, les alginates et la gomme gellane.

Selon une variante de réalisation, le polysaccharide est de la gomme gellane, qui est un polysaccharide polyanionique de très haut poids moléculaire (au moins 500 000 daltons) fabriqué par des microorganismes. Solubilisé dans l'eau, le gellane donne une solution de viscosité faible avec laquelle il est possible de réaliser une émulsion par dispersion dans une phase hydrophobe. Lorsqu'une telle émulsion est chauffée à une température supérieure à 75°C et que des cations sont ajoutés par un procédé selon l'invention, les gouttelettes de la phase dispersée subissent une gélification. Après retour à la température ambiante, il est possible de récupérer les sphères ainsi formées et de les réticuler ultérieurement de façon chimique si nécessaire.

Selon une caractéristique avantageuse du procédé selon l'invention, la concentration de la solution de gellane est comprise entre 0,1 et 5 % et encore mieux voisine de 0,6 % (p/v).

Selon une autre caractéristique du procédé de fabrication, on réalise une solution éthanolique contenant des ions calcium ou magnésium ou sodium ou potassium de façon à amener la solution à une concentration en ions comprise entre 1 et 400 mM, de préférence plutôt comprise entre 5 et 10 mM, pour les ions calcium et magnésium et de préférence comprise entre 150 et 250 mM pour les ions sodium et potassium.

Selon une autre variante de réalisation, le polysaccharide utilisé est un carraghénane kappa ou un carraghénane iota qui gélifie respectivement en présence d'ions potassium ou calcium.

Selon une caractéristique avantageuse du procédé selon l'invention, on dissout le carraghénane à froid, à pH neutre ou alcalin dans des concentrations comprises entre 0,1 et 5 % (p/v), de préférence voisines de 1,5 %. On élève la température autour de 80°C, puis on réalise une émulsion par dispersion dans un liquide hydrophobe. On provoque alors la gélification des gouttelettes par addition d'une solution alcoolique contenant des ions potassium ou calcium selon que la solution aqueuse contient du carraghénane kappa ou iota.

Selon une troisième variante de réalisation, le polysaccharide utilisé est une pectine faiblement méthylée qui forme des solutions gélifiables en présence d'ions calcium.

Selon une caractéristique avantageuse, on réalise une solution de pectine de concentration comprise entre 0,1 et 10 %, de préférence de l'ordre de 1 à 2 % (p/v), à froid, à pH neutre. On réalise une émulsion par dispersion dans un liquide hydrophobe. Puis on rajoute, selon l'invention, des ions calcium sous forme d'une solution alcoolique de chlorure de calcium.

Selon une dernière variante de réalisation, on réalise le même type de procédé à partir d'une solution contenant de 1 à 5 % (p/v) d'alginate de sodium.

Enfin, selon encore un autre aspect, la présente invention concerne encore des microparticules, en particulier des microcapsules, caractérisées en ce qu'elles sont réalisées à partir de substances ou de mélanges de substances comprenant des groupements carboxyliques, des groupements aminés et/ou des fonctions alcools qui sont réticulés suite à l'activation des groupements carboxyliques par un agent d'activation ne réalisant pas de pontage, de façon à former des liaisons amides avec les groupements aminés et/ou des liaisons ester avec les fonctions alcools.

L'invention concerne aussi des microparticules ou des microcapsules, caractérisées en ce que les microparticules comprennent dans leur masse le produit de réaction entre un polysaccharide porteur de groupements carboxyliques estérifiés, et soit une substance polyaminée comme par exemple une protéine, soit une substance polyhydroxylée comme par exemple un polysaccharide;

ledites microcapsules présentent une paroi constituée du produit de réaction entre un polysaccharide porteur de groupements carboxyliques estérifiés, et soit une substance polyaminée comme par exemple une protéine, soit une substance polyhydroxylée comme par exemple un polysaccharide.

Les caractéristiques avantageuses de ces microparticules, en particulier des microcapsules, font également l'objet de sous-revendications qui sont incorporées ici par référence et qui résultent de la description du procédé.

L'invention concerne encore une composition telle qu'une composition cosmétique ou une composition pharmaceutique, ou une composition alimentaire, caractérisée en ce qu'elle comprend des microparticules, en particulier des microcapsules, telles que définies ci-dessus, ou obtenues par le procédé précité.

D'autres buts, caractéristiques et avantages de l'invention apparaîtront clairement à la lumière de la description explicative qui va suivre, faite en référence à plusieurs exemples de réalisation de l'invention donnés simplement à titre d'illustration et qui ne sauraient donc en aucune façon limiter la portée de l'invention. Dans les exemples, les pourcentages sont donnés en poids sauf indication contraire.

### Exemple 1 de l'invention

### Fabrication de microparticules d'atélocollagène

### a) Préparation de la phase aqueuse

On prépare une solution d'atélocollagène bovin à 2% (p/v) dont le pH est ajusté à 4.

### b) Emulsification

On émulsionne 40 ml de cette phase aqueuse dans 250 ml de cocoate d'éthyle-2-hexyle contenant 2 % v/v de Span 85®, ICI, comme phase dispersante, par agitation mécanique pendant quelques minutes.

### c) Alcalinisation

On ajoute deux fois 10 ml de soude à 2 % (p/v) dans de l'éthanol à 95 % à 10 min d'intervalle. On laisse la réaction se dérouler pendant 10 min après chaque ajout.

### d) Lavages

Les microparticules sous forme de sphères sont recueillies par centrifugation, puis lavées dans plusieurs bains d'éthanol à 95 %.

### e) Réticulation

Les sphères sont réticulées par incubation pendant 24 h à 4°C dans un bain de diméthylformamide contenant 0,5 % (v/v) de diphénylphosphorylazide, puis pendant 4 h à température ambiante dans du tampon borate pH 8,9 (tétraborate de sodium 0,04 M, acide borique (0,04 M).

Après lavages à l'eau, les microparticules ainsi obtenues peuvent être lyophilisées.

### Exemple 2 de l'invention

### Fabrication de microparticules de chitosan

### a) Préparation de la phase aqueuse

On prépare une solution à 1 % (p/v) de chitosan HPM hautement désacétylé (Aber Technologies) dans de l'acide acétique à 1,25 %, pH 4.

### b) Emulsification

On réalise une émulsion par dispersion de 50 ml de cette solution dans 250 ml de myristate d'isopropyle contenant 2 % (v/v) de Span 85®. L'émulsion est maintenue sous agitation mécanique pendant 10 min.

### c) Alcalinisation

On ajoute 16 ml de soude à 2 % p/v dans l'éthanol à 95 % et on poursuit l'agitation pendant 15 min.

### d) Lavages

Les microparticules sont récupérées par centrifugation et lavées dans plusieurs bains d'éthanol, puis d'eau.

### e) Réticulation

Les microparticules peuvent être réticulées selon l'exemple 1.

### Exemple 3 selon l'invention

### Fabrication de microparticules de diamètre moyen 150 µm à partir de sérumalbumine humaine (HSA) et d'alginate de propylèneglycol (PGA).

### a) Préparation de la phase aqueuse

On prépare, par agitation magnétique de 10 min à température ambiante, une solution dans l'eau distillée renfermant 20 % d'HSA (Centre de Transfusion Sanguine, Strasbourg) et 1 % d'un PGA présentant un taux d'estérification compris entre 80 et 85 % (Kelcoloïd S®, KELCO International).

### b) Emulsification

On émulsionne 6 ml de cette phase aqueuse utilisée comme phase dispersée dans 40 ml de myristate d'isopropyle contenant 2 % v/v de Span 85® comme phase dispersante, par agitation mécanique de 5 min à 2 000 tr/min.

### c) Alcalinisation

On ajoute à l'émulsion en agitation 2 ml d'une solution de soude à 2 % p/v dans l'éthanol à 95 % et on laisse la réaction de transacylation se développer pendant 15 min, ce qui produit des microparticules.

### d) Acidification

On ajoute au milieu réactionnel en agitation 2 ml d'une solution à 7,6 % v/v d'acide acétique dans l'éthanol à 95 %. L'agitation est encore maintenue pendant 15 min de manière à permettre la neutralisation des microparticules formées.

### e) Lavages

Les microparticules sont séparées par centrifugation, puis lavées par remises en suspension dans de l'éthanol à 95 % renfermant 2 % de Tween 20®, ICI, puis dans l'éthanol à 95 %, puis dans l'eau distillée.

Les microparticules peuvent être ensuite congelées et lyophilisées.

On obtient des sphères transparentes de taille moyenne 150 µm. Après lyophilisation, la réhydratation de la poudre obtenue montre que les microparticules sont intactes et reprennent leur forme sphérique.

### Essais de stabilité dans divers milieux contenant ou non des protéases

Dans des tubes à essais, des prises d'essai de 25 mg de microparticules lyophilisées sont réhydratées par addition de 1 ml d'eau distillée, puis additionnées de 7,5 ml de différents milieux :
- de l'eau distillée
- une solution de pH acide (1,2) additionnée ou non de pepsine (milieu gastrique artificiel, USP XXI)
- une solution de pH légèrement alcalin (7,5), additionnée ou non de trypsine (0,25 % p/v).

Les tubes sont incubés à 37°C. La stabilité des microparticules est étudiée par examen microscopique. Le temps de lyse est le temps au bout duquel toutes les microparticules ont disparu du milieu.

### Résultats

Les microparticules préparées selon cet exemple sont stables plus de 3 j dans l'eau distillée comme dans les solutions de pH 1,2 ou de pH 7,5. Elles sont dégradées par les protéases : en 15 min par la pepsine, en 25 min par la trypsine.

### Exemple 4 de l'invention

### Fabrication de microparticules à partir de gélatine et de PGA.

### Préparation de la phase aqueuse

On prépare, à la température de 40°C, 8 ml d'une solution aqueuse de gélatine type B, bloom 150, à la concentration de 10 %, et de PGA à la concentration de 1 %.

### Emulsification

Dans un récipient thermostaté à 40°C, 6 ml de cette phase aqueuse sont émulsionnés dans 40 ml de myristate d'isopropyle contenant 2 % de Span 85 et préchauffés à la température de 40°C (vitesse d'agitation : 2 000 tr/min).

### L'alcalinisation

La neutralisation et les lavages sont ensuite effectués comme décrit dans l'exemple 3. Les microparticules apparaissent comme des sphères de diamètre moyen 1 mm. Après lyophilisation, elles donnent une poudre blanche qui se réhydrate facilement.

### Exemple 5 de l'invention

### Fabrication de microparticules à partir d'atélocollagène et chondroïtine sulfate et de PGA.

### Préparation de la phase aqueuse

Une solution à 1,6 % d'atélocollagène et 0,6 % de chondroïtine sulfate dans un tampon phosphate pH 7,4 est additionnée de PGA à la concentration de 0,7 %. Le protocole décrit à l'exemple 3 est ensuite appliqué à cette solution aqueuse.

On obtient des microparticules de diamètre moyen 600 µm.

### Exemple 6 de l'invention

### Fabrication de microparticules à partir d'un concentré de protéines du lactosérum et de PGA.

### Préparation de la phase aqueuse

Dans 16 ml d'eau distillée, on dissout 160 mg de PGA, et 3,2 g de concentré de protéines du lactosérum (Prosobel S65E®, Bel Industries).

Le protocole décrit à l'exemple 3 est ensuite reproduit en utilisant comme phase aqueuse 12 ml de la solution précédente et en doublant tous les volumes des différents réactifs.

On obtient des microparticules sphériques à contenu granuleux, de diamètre moyen 500 µm, qui sont intactes après lyophilisation.

### Exemple 7 de l'invention

### Fabrication de microparticules à partir d'ovalbumine et de pectine.

### Préparation de la phase aqueuse

Dans 8 ml d'eau distillée, on dissout 240 mg de pectine de pomme (FLUKA, estérification : 70 à 75 %), et 800 mg d'ovalbumine.

Puis le protocole décrit dans l'exemple 3 est appliqué, en utilisant 6 ml de la solution précédente, en remplaçant le myristate d'isopropyle par de l'huile de paraffine fluide, et en doublant les volumes des solutions alcaline et acide.

On obtient des microparticules de taille moyenne 200 µm, à contenu granuleux.

### Exemple 8 de l'invention

Fabrication de microparticules à partir de carboxyméthylcellulose (CMC) et de PGA.

Préparation de la phase aqueuse : dans 8 ml d'eau distillée, on dissout 80 mg de CMC (CMC 7 LF, degré de substitution : 0,7, HERCULES) et 320 mg de PGA, par agitation magnétique de 15 min à 40°C.

Puis le protocole décrit dans l'exemple 3 est appliqué, en utilisant 6 ml de la solution précédente.

On obtient des microparticules granuleuses de taille moyenne 1,8 mm.

### Exemple 9 de l'invention

### Fabrication de microparticules d'atélocollagène.

### a) Préparation de la phase aqueuse

On prépare une solution d'atélocollagène bovin à 2 % (p/v) dont le pH est ajusté à 3,7 comme il est bien connu à l'homme de l'art notamment à partir de documents précédents du déposant tel que EP-A-0318154 auquel l'homme de l'art pourra se reporter.

### b) Emulsification

On émulsionne 50 ml de cette phase dans 200 ml de cocoate d'éthyle-2-hexyle comme phase dispersante, par agitation mécanique pendant quelques minutes.

### c) Addition du non-solvant

On ajoute 400 ml d'alcool éthylique à 95° sans arrêter l'agitation. On stoppe l'agitation au bout de quelques secondes ce qui provoque la précipitation de microparticules.

### d) Lavage

Les microparticules obtenues sont recueillies par filtration , puis lavées dans plusieurs bains d'éthanol à 95°.

### e) Réticulation

Les microparticules sont de préférence réticulées par incubation pendant 24 h à 4°C dans un bain de diméthylformamide contenant 0,5 % (v/v) de diphénylphosphorylazide, puis pendant 4 h à température ambiante dans du tampon borate pH 8,9.

Après lavage à l'eau, les microparticules ainsi obtenues peuvent être lyophilisées.

### Exemple 10 de l'invention

### Fabrication de microparticules d'atélocollagène.

Dans cet exemple, on procède comme décrit à l'exemple 9 si ce n'est que l'on remplace l'alcool éthylique par l'acétone comme non solvant pour précipiter les microparticules.

Après plusieurs lavages des microparticules dans plusieurs bains d'acétone, on peut réaliser la réticulation comme décrit à l'exemple 9.

Après lavage à l'eau, les microparticules peuvent aussi être lyophilisées.

## Revendications

1. Procédé de fabrication de microparticules, en particulier de microcapsules, caractérisé en ce que l'on prépare tout d'abord une solution essentiellement homogène d'une substance ou d'un mélange de substances dans un solvant, que l'on réalise une émulsion de la solution dans un liquide dispersant formant une phase continue dans lequel ladite substance ou ledit mélange sont essentiellement insolubles, et formant une phase dispersée puis on déclenche une réaction chimique ou physicochimique dans la phase dispersée par modification de la composition chimique in situ de ladite substance ou dudit mélange dans la phase dispersée, en ajoutant un agent essentiellement non soluble ou non miscible dans ou à la phase continue, dans les conditions d'addition, en provoquant ainsi une modification de l'état physicochimique résultant en une insolubilisation de la substance ou du mélange de substances et en l'individualisation desdites microparticules, en présence du solvant de départ, lesdites microparticules étant ensuite récupérées.

2. Procédé selon la revendication 1, caractérisé en ce que l'agent essentiellement non soluble ou non miscible dans ou à la phase continue, dans les conditions d'addition, que l'on souhaite incorporer à la phase dispersée est présent à l'état dissous dans un solvant miscible au solvant de la phase dispersée et présentant une affinité pour le liquide dispersant de la phase continue inférieure à l'affinité vis-à-vis de la phase dispersée.

3. Procédé selon la revendication 1 ou 2, caractérisé en ce qu'on utilise comme substance ou mélange de substances précitées une substance choisie parmi des acides nucléiques, une protéine, ou un polysaccharide, ou les divers mélanges de ces substances.

4. Procédé selon la revendication 1 à 3, caractérisé en ce que comme réaction chimique, on réalise la formation de liaisons covalentes en particulier des estérifications et des amidations ou la formation de liaisons ioniques entre les groupements ionisables d'une substance ou d'un mélange de substances.

5. Procédé selon l'une des revendications 1 à 3, caractérisé en ce que comme réaction physicochimique, on réalise une réaction choisie parmi une réaction de coacervation, de précipitation ou de désolvatation.

6. Procédé selon l'une des revendications 1 à 5, caractérisé en ce que l'agent essentiellement non soluble ou non miscible précité dans ou à la phase continue conduit à une séparation de phase au sein de la solution initialement homogène de la phase dispersée, ou à une gélification de ladite solution de la phase dispersée ou à une perte de solubilité de la phase dispersée par condensation ou par polymérisation.

7. Procédé selon l'une des revendications 1 à 3 ou 6, caractérisé en ce que l'agent essentiellement non soluble ou non miscible précité dans ou à la phase continue, dans les conditions d'addition, est un non solvant pour la substance ou le mélange de substances solubilisée(s) dans le solvant de la phase dispersée, ou ledit agent conduit à une modification de pH, ou ledit agent comprend au moins un électrolyte, ou ledit agent comprend au moins une molécule susceptible de réagir avec la ou les substances dissoutes dans la phase dispersée de l'émulsion.

8. Procédé selon l'une des revendications 1 à 3 ou 5 à 7, caractérisé en ce que le non solvant pour la substance ou le mélange de substances solubilisée(s) dans le solvant de la phase dispersée, et essentiellement non miscible à la phase continue dans les conditions d'addition, provoque une insolubilisation de la phase dispersée, en particulier une gélification ou une coagulation.

9. Procédé selon l'une des revendications 5 à 8, caractérisé en ce que le non solvant est choisi parmi un alcool, en particulier un alcool inférieur en C₁-C₆, de préférence l'alcool éthylique, ou une cétone, en particulier une cétone inférieure en C₂-C₆, encore de préférence l'acétone.

10. Procédé selon l'une des revendications précédentes, caractérisé en ce qu'on réalise les étapes successives suivantes :
a) on prépare une solution aqueuse d'une substance choisie parmi les acides nucléiques, une protéine ou un polysaccharide ou les divers mélanges de ces substances ;
b) on prévoit un liquide hydrophobe dans lequel la substance ou les substances précitées sont essentiellement insolubles ;
c) on mélange le liquide hydrophobe et la phase aqueuse pour former une émulsion dans laquelle la solution aqueuse est la phase dispersée et le liquide hydrophobe est la phase continue ;
d) on ajoute à l'émulsion le non solvant de la substance solubilisée dans la phase dispersée, dans des proportions telles que ledit non solvant soit essentiellement non miscible à la phase continue en formant ainsi des microparticules par gélification ou coagulation ;
e) on recueille par des moyens de séparation physique, par exemple par filtration, centrifugation ou décantation, les microparticules formées par ladite gélification ou ladite coagulation ;
f) de préférence, on renforce la cohésion des microparticules par une réticulation.

11. Procédé selon l'une des revendications 1 à 3 ou 7, caractérisé en ce qu'on réalise tout d'abord une émulsion d'une solution aqueuse d'une substance ou d'un mélange de substances susceptibles de subir par réaction chimique ou physicochimique une variation d'état physicochimique traduite par une insolubilisation provoquée par une modification de pH, à un pH auquel l'état physicochimique de ladite substance ou dudit mélange de substances est celui d'une solution de viscosité appropriée à la réalisation d'une émulsion ; puis on fait varier le pH, en ajoutant l'agent non soluble ou non miscible précité, comprenant une substance modifiant le pH dissoute dans un solvant organique miscible à la phase aqueuse, pour déclencher la réaction chimique ou physicochimique et amener la substance ou le mélange de substances dans un état physicochimique correspondant à une insolubilisation de la substance ou du mélange et qui conduit à la formation de microparticules physiquement individualisées.

12. Procédé selon la revendication 11, caractérisé en ce qu'on utilise comme substance modifiant le pH un acide, une base ou un tampon en fonction de la valeur de pH souhaitée.

13. Procédé selon la revendication 11 ou 12, caractérisé en ce qu'on réalise les étapes successives suivantes :
a) on prépare une solution aqueuse d'une substance choisie parmi les acides nucléiques, un polysaccharide ou une protéine ou les divers mélanges de ces substances, le pH étant choisi de façon que la substance ou le mélange de substances forme une solution essentiellement homogène de viscosité compatible avec la réalisation d'une émulsion ;
b) on prévoit un liquide hydrophobe dans lequel la substance ou les substances précitées sont essentiellement insolubles ;
c) on mélange le liquide hydrophobe et la phase aqueuse pour former une émulsion ;
d) on ajoute à l'émulsion soit une solution d'une substance alcaline dans un liquide organique miscible à la phase aqueuse si l'on souhaite alcaliniser la phase aqueuse ou une solution d'une substance acide dans un liquide organique miscible à la phase aqueuse si l'on souhaite acidifier la phase aqueuse ;
e) après une période de temps prédéterminée nécessaire pour réaliser la coacervation de la ou des substances initialement solubilisées, on recueille les microparticules formées par centrifugation ou par filtration ;
f) de préférence, on renforce la cohésion des microparticules par une réticulation.

14. Procédé selon la revendication 13, caractérisé en ce qu'on utilise comme agent de réticulation des composés polyaminés, polycarboxylés ou polyhydroxylés précités ou de leur mélange, un agent bifonctionnel tel que le formaldéhyde, le glutaraldéhyde, et les di-aldéhydes, les dichlorures d'acides, les di-anhydrides d'acides, les di-isocyanates, les di-imidoesters, les bis-chloroformiates, les succinimides ; ou un agent d'activation de groupements carboxyliques pour former des liaisons amides avec les groupements aminés ou des liaisons esters avec les fonctions alcools de la substance ou du mélange de substances, tel qu'un carbodiimide ou un azide.

15. Procédé selon l'une quelconque des revendications précédentes, caractérisé en ce qu'on utilise une protéine de préférence choisie parmi le collagène ou un dérivé de collagène, de préférence l'atélocollagène ; ou un polysaccharide de préférence choisi parmi un glycosaminoglycanne ou le chitosan.

16. Procédé selon l'une des revendications 1 à 4 et 15, caractérisé en ce qu'on réalise une réaction chimique de transacylation qui se produit à pH alcalin entre un polysaccharide porteur de groupements carboxyliques estérifiés et soit une substance polyaminé comme par exemple une protéine, soit une substance polyhydroxylé comme par exemple un polysaccharide porteur de groupement hydroxyles.

17. Procédé selon la revendication 16, caractérisé en ce qu'il comprend :
a) on prépare une solution aqueuse neutre donc non réactive renfermant d'une part un polysaccharide porteur de groupements carboxyliques estérifiés, et d'autre part soit une substance polyaminée comme par exemple une protéine, soit une substance polyhydroxylée comme par exemple un polysaccharide porteur de groupements hydroxyles ;
b) on prévoit un liquide hydrophobe dans lequel le polysaccharide estérifié et la substance polyaminée ou polyhydroxylée sont essentiellement insolubles ;
c) on mélange le liquide hydrophobe et la solution aqueuse pour former une émulsion ;
d) on ajoute à l'émulsion une solution d'une substance alcaline dans un liquide organique miscible à la phase aqueuse, ce qui permet l'obtention de conditions physico-chimiques favorables au déroulement de la réaction entre la substance polyaminée ou la substance polyhydroxylée et le polysaccharide porteur des groupements carboxyliques estérifiés ;
e) après une période de temps prédéterminée nécessaire pour réaliser une réaction de transacylation, en formant ainsi des microparticules, en particulier des microcapsules, on réalise une neutralisation de l'émulsion, de préférence en ajoutant à l'émulsion une solution d'une substance acide dans un liquide organique miscible à la phase aqueuse ; ce qui neutralise et stabilise les microparticules, en particulier les microcapsules formées.

18. Procédé selon la revendication 17, caractérisé en ce qu'on forme une émulsion de la solution aqueuse comme phase dispersée dans le liquide hydrophobe formant la phase continue.

19. Procédé selon l'une des revendications 1 à 18, caractérisé en ce qu'on utilise comme substance un polysaccharide capable de s'associer spécifiquement avec un ion ou un polyion pour former un gel, de préférence choisi parmi le carraghénane kappa et iota, une pectine faiblement méthylée, un alginate et la gomme gellane ayant de préférence un haut poids moléculaire d'au moins 500 000 daltons.

20. Procédé selon la revendication 19, caractérisé en ce qu'on utilise du gellane à une concentration en solution aqueuse comprise entre 0,1 et 5 % et encore mieux voisine de 0,6 % (p/v).

21. Procédé selon la revendication 19 ou 20, caractérisé en ce qu'on réalise une solution éthanolique contenant des ions calcium ou magnésium ou sodium ou potassium de façon à amener la solution à une concentration en ions comprise entre 1 et 400 mM.

22. Procédé selon la revendication 19, caractérisé en ce qu'on utilise du carraghénane kappa ou iota qui se gélifie respectivement en présence d'ions potassium ou calcium ; on dissout le carraghénane à froid, à pH neutre ou alcalin dans des concentrations comprises entre 0,1 et 5 % (p/v), on élève la température autour de 80°C et on réalise une émulsion par dispersion dans un liquide hydrophobe ; on provoque ensuite la gélification des gouttelettes par addition d'une solution alcoolique contenant des ions potassium ou calcium selon que la solution aqueuse contienne du carraghénane kappa ou iota.

23. Procédé selon la revendication 19, caractérisé en ce qu'on utilise comme polysaccharide une pectine faiblement méthylée qui forme des solutions gélifiables en présence d'ions calcium ; la solution de pectine étant réalisée à une concentration comprise entre 0,1 et 10 % (p/v) à froid, à pH neutre, puis on réalise une émulsion par dispersion dans un liquide hydrophobe, puis on rajoute enfin des ions calcium sous forme d'une solution alcoolique de chlorure de calcium.

24. Procédé selon la revendication 19, caractérisé en ce qu'on utilise comme polysaccharide une solution d'alginate de sodium à 1 à 5 % (p/v).

25. Microparticules, en particulier microcapsules, caractérisées en ce qu'elles sont réalisées à partir de substances ou de mélanges de substances comprenant des groupements carboxyliques, des groupements aminés et/ou des fonctions alcools qui sont réticulés suite à l'activation des groupements carboxyliques par un agent d'activation qui ne réalisent pas de pontage de façon à former des liaisons amides avec les groupements aminés et/ou es liaisons ester avec les fonctions alcools.

26. Microparticules ou microcapsules, caractérisées en ce que les microparticules comprennent dans leur masse le produit de réaction entre un polysaccharide porteur de groupements carboxyliques estérifiés et soit une substance polyaminée comme par exemple une protéine, soit une substance polyhydroxylée comme par exemple un polysaccharide; lesdites microcapsules présentent une paroi constituée du produit de réaction entre un polysaccharide porteur de groupements carboxyliques estérifiés, et soit une substance polyaminée comme par exemple une protéine, soit une substance polyhydroxylée comme par exemple un polysaccharide.

27. Microparticules, en particulier microcapsules selon l'une des revendications 25 ou 26, caractérisées en ce que la ou les substances précitées sont choisies parmi le groupe consistant d'une protéine, d'un polysaccharide ou d'un acide nucléique, des mélanges de protéines, des mélanges de polysaccharides ou des mélanges de protéines ou de polysaccharides, éventuellement avec des acides nucléiques.

28. Microparticules, en particulier microcapsules selon l'une des revendications 25 ou 27, caractérisées en ce que les protéines sont choisies parmi le groupe consistant de α-lactalbumine, β-lactoglobuline, caséines, ovalbumine, albumines animales, globulines sanguines, hémoglobine, fibrinogène, collagène, atélocollagène, gélatine, kératine, albumines végétales, globulines végétales, gluténines, gliadine; les extraits protéiques issus du lait, de la soie, des céréales, des légumineuses, des algues, du poisson; le polysaccharide précité est choisi parmi le groupe consistant de l'agar, l'agarose, l'agaropectine les carraghénanes, les alginates, les pectines, l'amylose, l'amylopectine, l'amidon, les amidons modifiés, les galactomannanes tels que guar, caroube, le glucomannane, le konjac, les celluloses modifiées, l'inuline, le xanthane, le dextrane, le curdlane, la gellane, le chitosan, les chondroïtines sulfates, l'acide hyaluronique, le dermatane sulfate, l'héparane sulfate, l'héparine, le kératane sulfate; l'acide nucléique est choisi parmi l'acide ribonucléique et l'acide désoxyribonucléique.

29. Microparticules, en particulier microcapsules selon l'une des revendications 25 à 28, caractérisées en ce que le polysaccharide précité s'associe specifiquement avec des cations ou polycations pour former un gel.

30. Microparticules, en particulier microcapsules selon la revendication 29, caractérisées en ce que le polysaccharide précité est choisi parmi le groupe consistant d'un carraghénane kappa ou iota, d'une pectine faiblement méthylée, d'un alginate et la gomme gellane.

31. Microparticules ou microcapsules selon l'une des revendications 25 à 30, caractérisées en ce que le polysaccharide estérifié est un alginate de propylèneglycol ou une pectine faiblement methylée qui forme une solution gelifiable en présence d'ions calcium.

32. Microparticules ou microcapsules selon l'une des revendications 25 à 31, caractérisées en ce que la substance polyaminée ou polyhydroxylée précitée est choisie parmi la sérum-albumine humaine, la gélatine, les protéines du lactoserum, l'ovalbumine, un melange atélocollagène et chondroïtine sulfate ou la carboxyméthylcellulose.

33. Microparticules, en particulier microcapsules, selon l'une des revendications 25 à 32, caractérisées en ce qu'elles sont lyophilisées.

34. Composition telle qu'une composition cosmétique ou une composition pharmaceutique ou une composition alimentaire, caractérisée en qu'elle comprend des microparticules, en particulier des microcapsules, telles que définies dans l'une quelconque des revendications 25 à 33, ou telles qu'obtenues par le procédé selon l'une quelconque des revendications 1 à 24.

## Claims

1. A process for the manufacture of microparticles, in particular microcapsules, characterised in that an essentially homogeneous solution of a substance or mixture of substances in a solvent is first prepared, an emulsion of the solution is produced in a dispersing liquid forming a continuous phase, in which said substance or said mixture is essentially insoluble, and forming a disperse phase, and a chemical or physicochemical reaction is then initiated in the disperse phase by modification of the *in situ* chemical composition of said substance or said mixture in the disperse phase through the addition of an agent which is essentially insoluble in or immiscible with the continuous phase, under the conditions of addition, thereby modifying the physicochemical state resulting in the insolubilization of the substance or mixture of substances and in the individualization of said microparticles, in the presence of the starting solvent, said microparticles then being recovered.

2. A process according to claim 1, characterised in that the agent which is essentially insoluble in or immiscible with the continuous phase, under the conditions of addition, and which it is desired to incorporate into the disperse phase is present as a solution in a solvent which is miscible with the solvent of the disperse phase and which has a lower affinity for the dispersing liquid of the continuous phase than for the disperse phase.

3. A process according to claim 1 or 2, characterised in that a substance selected from nucleic acids, a protein or a polysaccharide, or various mixtures of these substances, is used as the abovementioned substance or mixture of substances.

4. A process according to claims 1 to 3, characterised in that the formation of covalent bonds, in particular esterifications and amidations, or the formation of ionic bonds between the ionizable groups of a substance or mixture of substances is effected as the chemical reaction.

5. A process according to one of claims 1 to 3, characterised in that a reaction selected from a coacervation, precipitation or insolubilization reaction is effected as the physicochemical reaction.

6. A process according to one of claims 1 to 5, characterised in that the abovementioned agent which is essentially insoluble in or immiscible with the continuous phase causes a phase separation within the initially homogeneous solution of the disperse phase, the gelling of said solution of the disperse phase or a loss of solubility of the disperse phase by condensation or polymerization.

7. A process according to one of claims 1 to 3 or 6, characterised in that the abovementioned agent which is essentially insoluble in or immiscible with the continuous phase, under the conditions of addition, is a non-solvent for the substance or mixture of substances solubilized in the solvent of the disperse phase, or said agent causes a pH modification, or said agent comprises at least one electrolyte, or said agent comprises at least one molecule capable of reacting with the substance or substances dissolved in the disperse phase of the emulsion.

8. A process according to one of claims 1 to 3 or 5 to 7, characterised in that the non-solvent for the substance or mixture of substances solubilized in the solvent of the disperse phase, which is essentially immiscible with the continuous phase under the conditions of addition, causes insolubilization of the disperse phase, in particular gelling or coagulation.

9. A process according to one of claims 5 to 8, characterised in that the non-solvent is selected from an alcohol, in particular a C₁-C₆ lower alcohol, preferably ethyl alcohol, or a ketone, in particular a C₂-C₆ lower ketone, preferably acetone.

10. A process according to one of the preceding claims, characterised in that the following successive steps are performed:
a) an aqueous solution of a substance selected from nucleic acids, a protein or a polysaccharide, or various mixtures of these substances, is prepared;
b) a hydrophobic liquid is provided in which the abovementioned substance or substances are essentially insoluble;
c) the hydrophobic liquid and the aqueous phase are mixed to form an emulsion in which the aqueous solution is the disperse phase and the hydrophobic liquid is the continuous phase;
d) the non-solvent for the substance solubilized in the disperse phase is added to the emulsion in proportions such that said non-solvent is essentially immiscible with the continuous phase, thereby forming microparticles by gelling or coagulation;
e) the microparticles formed by said gelling or said coagulation are collected by physical separation means, for example by filtration, centrifugation or decantation; and
f) the cohesion of the microparticles is preferably strengthened by crosslinking.

11. A process according to one of claims 1 to 3 or 7, characterised in that the first step is to produce an emulsion of an aqueous solution of a substance or mixture of substances capable of undergoing, by a chemical or physicochemical reaction, a variation in physicochemical state represented by insolubilization caused by a pH modification, at a pH at which the physicochemical state of said substance or said mixture of substances is that of a solution whose viscosity is appropriate for the production of an emulsion; the pH is then varied by the addition of the abovementioned insoluble or immiscible agent, comprising a pH-modifying substance dissolved in an organic solvent miscible with the aqueous phase, in order to initiate the chemical or physicochemical reaction and bring the substance or mixture of substances into a physicochemical state which corresponds to insolubilization of the substance or mixture and which results in the formation of physically individualized microparticles.

12. A process according to claim 11, characterised in that the pH-modifying substance used is an acid, a base or a buffer, depending on the desired pH value.

13. A process according to claim 11 or 12, characterised in that the following successive steps are performed:
a) an aqueous solution of a substance selected from nucleic acids, a polysaccharide or a protein, or various mixtures of these substances, is prepared, the pH being chosen so that the substance or mixture of substances forms an essentially homogeneous solution whose viscosity is compatible with the production of an emulsion;
b) a hydrophobic liquid is provided in which the abovementioned substance or substances are essentially insoluble;
c) the hydrophobic liquid and the aqueous phase are mixed to form an emulsion;
d) either a solution of an alkaline substance in an organic liquid miscible with the aqueous phase is added to the emulsion, if it is desired to render the aqueous phase alkaline, or a solution of an acid substance in an organic liquid miscible with the aqueous phase is added to the emulsion, if it is desired to acidify the aqueous phase;
e) after a predetermined period of time required to effect the coacervation of the initially solubilized substance or substances, the microparticles formed are collected by centrifugation or filtration; and
f) the cohesion of the microparticles is preferably strengthened by crosslinking.

14. A process according to claim 13, characterised in that the agent used for crosslinking the abovementioned polyamino, polycarboxylic or polyhydroxylic compounds or a mixture thereof is a difunctional agent such as formaldehyde, glutaraldehyde and dialdehydes, acid dichlorides, acid dianhydrides, diisocyanates, diimidoesters, bischloroformates and succinimides, or an agent which activates carboxyl groups to form amide bonds with the amino groups or ester bonds with the alcohol groups of the substance or mixture of substances, such as a carbodiimide or an azide.

15. A process according to any one of the preceding claims, characterised in that the protein used is preferably selected from collagen or a collagen derivative, preferably atelocollagen, or the polysaccharide used is preferably selected from a glycosaminoglycan or chitosan.

16. A process according to one of claims 1 to 4 and 5, characterised in that a chemical transacylation reaction is carried out which takes place at alkaline pH between a polysaccharide carrying esterified carboxyl groups and either a polyamino substance, for example a protein, or a polyhydroxylic substance, for example a polysaccharide carrying hydroxyl groups.

17. A process according to claim 16, characterised in that it comprises the following steps:
a) a neutral, *i.e.* unreactive, aqueous solution is prepared which contains on the one hand a polysaccharide carrying esterified carboxyl groups and on the other hand either a polyamino substance, for example a protein, or a polyhydroxylic substance, for example a polysaccharide carrying hydroxyl groups;
b) a hydrophobic liquid is provided in which the esterified polysaccharide and the polyamino or polyhydroxylic substance are essentially insoluble;
c) the hydrophobic liquid and the aqueous solution are mixed to form an emulsion;
d) a solution of an alkaline substance in an organic liquid miscible with the aqueous phase is added to the emulsion, making it possible to obtain physicochemical conditions favorable to the reaction between the polyamino substance or the polyhydroxylic substance and the polysaccharide carrying esterified carboxyl groups; and
e) after a predetermined period of time required to effect a transacylation reaction, thereby forming microparticles, in particular microcapsules, the emulsion is neutralized, preferably by the addition, to the emulsion, of a solution of an acid substance in an organic liquid miscible with the aqueous phase, which neutralizes and stabilizes the microparticles formed, in particular the microcapsules formed.

18. A process according to claim 17, characterised in that the emulsion formed is an emulsion of the aqueous solution as the disperse phase in the hydrophobic liquid forming the continuous phase.

19. A process according to one of claims 1 to 18, characterised in that the substance used is a polysaccharide which is capable of associating specifically with an ion or a polyion to form a gel, said polysaccharide preferably being selected from kappa- and iota-carrageenan, a pectin with a low degree of methylation, an alginate and gellan gum preferably having a high molecular weight of at least 500,000 daltons.

20. A process according to claim 19, characterised in that gellan is used in aqueous solution at a concentration of between 0.1 and 5% and preferably of about 0.6% (w/v).

21. A process according to claim 19 or 20, characterised in that an ethanolic solution is produced which contains calcium, magnesium, sodium or potassium ions so that the solution is brought to an ion concentration of between 1 and 400 mM.

22. A process according to claim 19, characterised in that kappa- or iota-carrageenan, which gels in the presence of potassium or calcium ions respectively, is used; the carrageenan is dissolved in the cold, at neutral or alkaline pH, in concentrations of between 0.1 and 5% (w/v), the temperature is raised to around 80°C and an emulsion is produced by dispersion in a hydrophobic liquid; gelling of the droplets is then caused by the addition of an alcoholic solution containing potassium or calcium ions, according to whether the aqueous solution contains kappa- or iota-carrageenan.

23. A process according to claim 19, characterised in that the polysaccharide used is a pectin with a low degree of methylation, which forms solutions gellable in the presence of calcium ions, the pectin solution being produced at a concentration of between 0.1 and 10% (w/v) in the cold at neutral pH, an emulsion is then produced by dispersion in a hydrophobic liquid and, finally, calcium ions are then added in the form of an alcoholic solution of calcium chloride.

24. A process according to claim 19, characterised in that the polysaccharide used is a 1 to 5% (w/v) solution of sodium alginate.

25. Microparticles, in particular microcapsules, characterised in that they are produced from substances or mixtures of substances comprising carboxyl groups, amino groups and/or alcohol groups that are crosslinked by an activator following the activation of the carboxyl groups with no bridges being formed so as to form amide bonds with the amino groups and/or ester bonds with the alcohol groups.

26. Microparticles or microcapsules, characterised in that the microparticles comprise in bulk the reaction product between a polysaccharide carrying esterified carboxyl groups and either a polyamino substance, such as for example a protein, or a polyhydroxylic substance, such as for example a polysaccharide; said microcapsules have a wall consisting of the reaction product between a polysaccharide carrying esterified carboxyl groups and either a polyamino substance, such as for example a protein, or a polyhydroxylic substance, such as for example a polysaccharide.

27. Microparticles, in particular microcapsules according to one of claims 25 or 26, characterised in that the abovementioned substance or substances are chosen from the group consisting of a protein, a polysaccharide or a nucleic acid, mixtures of proteins, mixtures of polysaccharides or mixtures of proteins or polysaccharides, optionally with nucleic acids.

28. Microparticles, in particular microcapsules according to one of claims 25 or 27, characterised in that the proteins are chosen from the group consisting of α-lactalbumin, β-lactoglobulin, caseins, ovalbumin, animal albumins, blood globulins, hemoglobin, fibrinogen, collagen, atelocollagen, gelatin, keratin, vegetable albumins, vegetable globulins, glutenins and gliadin; the protein extracts derived from milk, silk, cereals, leguminous plants, algae and fish; the abovementioned polysaccharide is chosen from the group consisting of agar, agarose, agaropectin, carrageenans, alginates, pectins, amylose, amylopectin, starch, modified starches, galactomannans (such as guar, carob), glucomannan, konjac, modified celluloses, inulin, xanthan, dextran, curdlan, gellan, chitosan, chondroitin sulfates, hyaluronic acid, dermatan sulfate, heparan sulfate, heparin, keratan sulfate; nucleic acid is chosen from ribonucleic acid and deoxyribonucleic acid.

29. Microparticles, in particular microcapsules according to one of claims 25 to 28, characterised in that the abovementioned polysaccharide associates specifically with cations or polycations to form a gel.

30. Microparticles, in particular microcapsules according to claim 29, characterised in that the abovementioned polysaccharide is chosen from the group consisting of a kappa- or iota-carrageenan, a pectin with a low degree of methylation, an alginate and a gellan gum.

31. Microparticles or microcapsules according to one of claims 25 to 30, characterised in that the esterified polysaccharide is a propylene glycol alginate or a pectin with a low degree of methylation, which forms a solution gellable in the presence of calcium ions.

32. Microparticles or microcapsules according to one of claims 25 to 31, characterised in that the abovementioned polyamino or polyhydroxylic substance is chosen from human serum albumin, gelatin, whey proteins, ovalbumin, an atelocollagen and chondroitin sulfate or carboxymethyl cellulose mixture.

33. Microparticles, in particular microcapsules, according to one of claims 25 to 32, characterised in that they are lyophilized.

34. A composition such as a cosmetic composition, a pharmaceutical composition or a food composition, characterised in that it comprises microparticles, in particular microcapsules, such as defined in any one of claims 25 to 33, or such as obtained by the process according to any one of claims 1 to 24.

## Patentansprüche

1. Verfahren zur Herstellung von Mikroteilchen, insbesondere Mikrokapseln, dadurch gekennzeichnet, daß zuerst eine im wesentlichen homogene Lösung einer Substanz oder einer Mischung von Substanzen in einem Lösungsmittel hergestellt wird, daß eine Emulsion der Lösung in einer Dispergierflüssigkeit erzeugt wird, indem eine kontinuierliche Phase gebildet wird, in der die Substanz oder die Mischung im wesentlichen unlöslich ist, und indem eine disperse Phase gebildet wird, und daß anschließend eine chemische oder physikochemische Reaktion in der dispersen Phase durch in situ-Modifikation der chemischen Zusammensetzung der Substanz oder der Mischung in der dispersen Phase ausgelöst wird, indem ein, unter den Zusatzbedingungen, im wesentlichen in der kontinuierlichen Phase nicht lösliches oder mit dieser nicht mischbares Mittel zugesetzt wird, wodurch eine Modifikation des physikochemischen Zustands hervorgerufen wird, die zu einer Insolubilisation der Substanz oder der Mischung von Substanzen und zur einzelnen Auftrennung der Mikroteilchen in Anwesenheit des Ausgangslösungsmittels führt, wobei die Mikroteilchen dann gewonnen werden.

2. Verfahren nach Anspruch 1, dadurch gekennzeichnet, daß das, unter den Zusatzbedingungen, im wesentlichen in der kontinuierlichen Phase nicht lösliche oder mit dieser nicht mischbare Mittel, das in die disperse Phase eingeschlossen werden soll, im gelösten Zustand in einem Lösungsmittel vorliegt, das mit dem Lösungsmittel der dispersen Phase mischbar ist und eine Affinität für die Dispergierflüssigkeit der kontinuierlichen Phase aufweist, die niedriger ist als die Affinität für die disperse Phase.

3. Verfahren nach Anspruch 1 oder 2, dadurch gekennzeichnet, daß als Substanz oder Mischung von Substanzen eine Substanz verwendet wird, die aus Nucleinsäuren, einem Protein, oder einem Polysaccharid, oder den verschiedenen Mischungen dieser Substanzen ausgewählt wird.

4. Verfahren nach Anspruch 1 bis 3, dadurch gekennzeichnet, daß als chemische Reaktion die Bildung kovalenter Bindungen, insbesondere Veresterungen und Amidierungen, oder die Bildung ionischer Bindungen zwischen den ionisierbaren Gruppen einer Substanz oder einer Mischung von Substanzen durchgeführt wird.

5. Verfahren nach einem der Ansprüche 1 bis 3, dadurch gekennzeichnet, daß als physikochemische Reaktion eine Reaktion durchgeführt wird, die aus einer Koazervation, Ausfällung oder Desolvatation ausgewählt wird.

6. Verfahren nach einem der Ansprüche 1 bis 5, dadurch gekennzeichnet, daß das in der kontinuierlichen Phase im wesentlichen nicht lösliche oder mit dieser nicht mischbare Mittel zu einer Phasentrennung in der anfänglich homogenen Lösung der dispersen Phase, oder zu einer Gelierung der Lösung der dispersen Phase oder zu einem Solubilitätsverlust der dispersen Phase durch Kondensation oder durch Polymerisation führt.

7. Verfahren nach einem der Ansprüche 1 bis 3 oder 6, dadurch gekennzeichnet, daß das, unter den Zusatzbedingungen, in der kontinuierlichen Phase im wesentlichen nicht lösliche oder mit dieser nicht mischbare Mittel ein Nicht-Lösungsmittel für die Substanz oder die Mischung von Substanzen ist, die im Lösungsmittel der dispersen Phase solubilisiert ist (sind), oder das Mittel zu einer Modifikation des pH führt, oder das Mittel zumindest einen Elektrolyten umfaßt, oder das Mittel zumindest ein Molekül umfaßt, das mit der oder den in der dispersen Phase der Emulsion gelösten Substanzen reagieren kann.

8. Verfahren nach einem der Ansprüche 1 bis 3 oder 5 bis 7, dadurch gekennzeichnet, daß das Nicht-Lösungsmittel für die Substanz oder die Mischung von Substanzen, die im Lösungsmittel der dispersen Phase solubilisiert ist (sind), welches unter den Zusatzbedingungen mit der kontinuierlichen Phase im wesentlichen nicht mischbar ist, eine Insolubilisation der dispersen Phase, insbesondere eine Gelierung oder Koagulation, hervorruft.

9. Verfahren nach einem der Ansprüche 5 bis 8, dadurch gekennzeichnet, daß das Nicht-Lösungsmittel aus einem Alkohol, insbesondere einem niederen C₁-C₆-Alkohol, vorzugsweise Ethylalkohol, oder einem Keton, insbesondere einem niederen C₂-C₆-Keton, vorzugsweise auch Aceton, ausgewählt wird.

10. Verfahren nach einem der vorhergehenden Ansprüche, dadurch gekennzeichnet, daß die folgenden aufeinanderfolgenden Schritte durchgeführt werden:
a) eine wässerige Lösung einer Substanz wird hergestellt, die aus Nucleinsäuren, einem Protein oder einem Polysaccharid oder den verschiedenen Mischungen dieser Substanzen ausgewählt wird;
b) eine hydrophobe Flüssigkeit wird vorgesehen, in der die Substanz oder die Substanzen im wesentlichen unlöslich sind;
c) die hydrophobe Flüssigkeit und die wässerige Phase werden gemischt, um eine Emulsion zu bilden, in der die wässerige Lösung die disperse Phase ist, und die hydrophobe Flüssigkeit die kontinuierliche Phase ist;
d) der Emulsion wird das Nicht-Lösungsmittel der in der dispersen Phase solubilisierten Phase in derartigen Mengen zugesetzt, daß das Nicht-Lösungsmittel mit der kontinuierlichen Phase im wesentlichen nicht mischbar ist, wobei so Mikroteilchen durch Gelierung oder Koagulation gebildet werden;
e) mit physikalischen Trennmitteln, beispielsweise durch Filtration, Zentrifugation oder Dekantieren, werden die durch die Gelierung oder Koagulation gebildeten Mikroteilchen gewonnen;
f) vorzugsweise wird die Kohäsion der Mikroteilchen durch eine Vernetzung verstärkt.

11. Verfahren nach einem der Ansprüche 1 bis 3 oder 7, dadurch gekennzeichnet, daß zuerst eine Emulsion einer wässerigen Lösung einer Substanz oder einer Mischung von Substanzen erzeugt wird, welche durch chemische oder physikochemische Reaktion eine Variation des physikochemischen Zustands eingehen können, die sich in einer Insolubilisation zeigt, welche durch eine Modifikation des pH auf einen pH hervorgerufen wird, bei dem der physikochemische Zustand der Substanz oder der Mischung von Substanzen jener einer Lösung mit einer zur Erzeugung einer Emulsion geeigneten Viskosität ist; und daß anschließend der pH variiert wird, indem das nicht lösliche oder nicht mischbare Mittel zugesetzt wird, das eine den pH modifizierende Substanz umfaßt, die in einem mit der wässerigen Phase mischbaren organischen Lösungsmittel gelöst ist, um die chemische oder physikochemische Reaktion auszulösen, und die Substanz oder die Mischung von Substanzen in einen physikochemischen Zustand zu bringen, der einer Insolubilisation der Substanz oder der Mischung von Substanzen entspricht, und der zur Bildung physisch einzeln aufgetrennter Teilchen führt.

12. Verfahren nach Anspruch 11, dadurch gekennzeichnet, daß als den pH modifizierende Substanz eine Säure, eine Base oder ein Puffer in Abhängigkeit vom gewünschten pH-Wert verwendet wird.

13. Verfahren nach Anspruch 11 oder 12, dadurch gekennzeichnet, daß die folgenden aufeinanderfolgenden Schritte durchgeführt werden:
a) eine wässerige Lösung einer Substanz wird hergestellt, die aus Nucleinsäuren, einem Polysaccharid oder einem Protein oder den verschiedenen Mischungen dieser Substanzen ausgewählt wird, wobei der pH derart ausgewählt wird, daß die Substanz oder die Mischung von Substanzen eine im wesentlichen homogene Lösung mit einer Viskosität bildet, die mit der Erzeugung einer Emulsion kompatibel ist;
b) eine hydrophobe Flüssigkeit wird vorgesehen, in der die Substanz oder die Substanzen im wesentlichen unlöslich sind;
c) die hydrophobe Flüssigkeit und die wässerige Phase werden gemischt, um eine Emulsion zu bilden;
d) der Emulsion wird entweder eine Lösung einer alkalischen Substanz in einer organischen Flüssigkeit, die mit der wässerigen Phase mischbar ist, zugesetzt, wenn die wässerige Phase alkalisch gemacht werden soll, oder eine Lösung einer sauren Substanz in einer organischen Flüssigkeit, die mit der wässerigen Phase mischbar ist, wenn die wässerige Phase angesäuert werden soll;
e) nach einem vorherbestimmten Zeitraum, der zur Durchführung der Koazervation der anfänglich solubilisierten Substanz oder Substanzen notwendig ist, werden die durch Zentrifugation oder Filtration gebildeten Mikroteilchen gewonnen;
f) vorzugsweise wird die Kohäsion der Mikroteilchen durch eine Vernetzung verstärkt.

14. Verfahren nach Anspruch 13, dadurch gekennzeichnet, daß als Vernetzungsmittel polyaminierter, polycarboxylierter oder polyhydroxylierter Verbindungen oder ihrer Mischungen ein bifunktionelles Mittel, wie Formaldehyd, Glutaraldehyd und Dialdeyhde, Säurechloride, Säuredianhydride, Diisocyanate, Diimidoester, Bis-chloroformiate, Succinimide; oder ein Aktivierungsmittel von Carboxyl-Gruppen zur Bildung von Amid-Bindungen mit den aminierten Gruppen oder von Ester-Bindungen mit den Alkohol-Funktionen der Substanz oder der Mischung von Substanzen, wie ein Carbodiimid oder ein Azid, verwendet wird.

15. Verfahren nach einem der vorhergehenden Ansprüche, dadurch gekennzeichnet, daß ein Protein, das vorzugsweise aus Kollagen oder einem Kollagen-Derivat, vorzugsweise Atelokollagen, ausgewählt wird; oder ein Polysaccharid, das vorzugsweise aus einem Glykosaminoglykan oder Chitosan ausgewählt wird, verwendet wird.

16. Verfahren nach einem der Ansprüche 1 bis 4 und 15, dadurch gekennzeichnet, daß eine chemische Transacylierungsreaktion durchgeführt wird, die bei einem alkalischen pH zwischen einem Polysaccharid, das veresterte Carboxyl-Gruppen trägt, und entweder einer polyaminierten Substanz, wie beispielsweise einem Protein, oder einer polyhydroxylierten Substanz, wie beispielsweise einem Polysaccharid, das Hydroxyl-Gruppen trägt, erfolgt.

17. Verfahren nach Anspruch 16, dadurch gekennzeichnet, daß es umfaßt:
a) eine neutrale, daher nicht reaktive wässerige Lösung wird hergestellt, die einerseits ein veresterte Carboxyl-Gruppen tragendes Polysaccharid und andererseits entweder eine polyaminierte Substanz, wie beispielsweise ein Protein, oder eine polyhydroxylierte Substanz, wie beispielsweise ein Hydroxyl-Gruppen tragendes Polysaccharid, enthält;
b) eine hydrophobe Flüssigkeit wird vorgesehen, in der das veresterte Polysaccharid und die polyaminierte oder polyhydroxylierte Substanz im wesentlichen unlöslich sind;
c) die hydrophobe Flüssigkeit und die wässerige Lösung werden gemischt, um eine Emulsion zu bilden;
d) der Emulsion wird eine Lösung einer alkalischen Substanz in einer mit der wässerigen Phase mischbaren organischen Flüssigkeit zugesetzt, wodurch das Erhalten physikochemischer Bedingungen ermöglicht wird, die für den Ablauf der Reaktion zwischen der polyaminierten Substanz oder der polyhydroxylierten Substanz und dem veresterte Carboxyl-Gruppen tragenden Polysaccharid vorteilhaft sind;
e) nach einem vorherbestimmten Zeitraum, der zur Durchführung einer Transacylierungsreaktion notwendig ist, wodurch Mikroteilchen, insbesondere Mikrokapseln, gebildet werden, wird eine Neutralisation der Emulsion vorgenommen, indem vorzugsweise der Emulsion eine Lösung einer sauren Substanz in einer mit der wässerigen Phase mischbaren organischen Flüssigkeit zugesetzt wird; wodurch die Mikroteilchen, insbesondere die gebildeten Mikrokapseln, neutralisiert und stabilisiert werden.

18. Verfahren nach Anspruch 17, dadurch gekennzeichnet, daß eine Emulsion der wässerigen Lösung als disperse Phase in der hydrophoben Flüssigkeit, welche die kontinuierliche Phase bildet, hergestellt wird.

19. Verfahren nach einem der Ansprüche 1 bis 18, dadurch gekennzeichnet, daß als Substanz ein Polysaccharid verwendet wird, das sich spezifisch mit einem Ion oder Polyion assoziieren kann, um ein Gel zu bilden, vorzugsweise ausgewählt aus Kappa- und Iota-Carrageenan, einem schwach methylierten Pektin, einem Alginat und Gellan-Gummi vorzugsweise mit einer hohen Molmasse von zumindest 500 000 Dalton.

20. Verfahren nach Anspruch 19, dadurch gekennzeichnet, daß Gellan bei einer Konzentration in wässeriger Lösung zwischen 0,1 und 5 % und bevorzugter von etwa 0,6 % (M/V) verwendet wird.

21. Verfahren nach Anspruch 19 oder 20, dadurch gekennzeichnet, daß eine ethanolische Lösung erzeugt wird, die Calcium- oder Magnesium- oder Natrium- oder Kaliumionen enthält, um die Lösung auf eine Ionenkonzentration zwischen 1 und 400 mM zu führen.

22. Verfahren nach Anspruch 19, dadurch gekennzeichnet, daß Kappa- oder Iota-Carrageenan verwendet wird, das jeweils in Anwesenheit von Kalium- oder Calciumionen geliert; das Carrageenan kalt bei neutralem oder alkalischem pH in Konzentrationen zwischen 0,1 und 5 % (M/V) gelöst wird, die Temperatur auf etwa 80°C erhöht wird, und eine Emulsion durch Dispersion in einer hydrophoben Flüssigkeit erzeugt wird; und anschließend die Gelierung von Tröpfchen durch den Zusatz einer alkoholischen Lösung hervorgerufen wird, die Kalium- oder Calciumionen enthält, je nachdem, ob die wässerige Lösung Kappa- oder Iota-Carrageenan enthält.

23. Verfahren nach Anspruch 19, dadurch gekennzeichnet, daß als Polysaccharid ein schwach methyliertes Pektin verwendet wird, das in Anwesenheit von Calciumionen gelierbare Lösungen bildet; wobei die Pektin-Lösung bei einer Konzentration zwischen 0,1 und 10 % (M/V) kalt bei neutralem pH gebildet wird, dann eine Emulsion durch Dispersion in einer hydrophoben Flüssigkeit erzeugt wird, und dann schließlich Calciumionen in Form einer alkoholischen Lösung von Calciumchlorid zugesetzt wird.

24. Verfahren nach Anspruch 19, dadurch gekennzeichnet, daß als Polysaccharid eine 1 bis 5 % (M/V) Natriumalginat-Lösung verwendet wird.

25. Mikroteilchen, insbesondere Mikrokapseln, dadurch gekennzeichnet, daß sie aus Substanzen oder Mischungen von Substanzen hergestellt werden, die Carboxyl-Gruppen, Amin-Gruppen und/oder Alkohol-Funktionen umfassen, welche nach der Aktivierung der Carboxyl-Gruppen durch ein Aktivierungsmittel vernetzt werden, und welche keine Brücken bilden, um Amid-Bindungen mit den Amin-Gruppen und/oder Ester-Bindungen mit den Alkohol-Funktionen zu bilden.

26. Mikroteilchen oder Mikrokapseln, dadurch gekennzeichnet, daß die Mikroteilchen in ihrer Masse das Reaktionsprodukt zwischen einem Polysaccharid, das veresterte Carboxyl-Gruppen trägt, und entweder einer polyaminierten Substanz, wie beispielsweise einem Protein, oder einer polyhydroxylierten Substanz, wie beispielsweise einem Polysaccharid, umfassen; wobei diese Mikrokapseln eine Wand aufweisen, die aus dem Reaktionsprodukt zwischen einem Polysaccharid, das veresterte Carboxyl-Gruppen trägt, und entweder einer polyaminierten Substanz, wie beispielsweise einem Protein, oder einer polyhydroxylierten Substanz, wie beispielsweise einem Polysaccharid, besteht.

27. Mikroteilchen, insbesondere Mikrokapseln, nach einem der Ansprüche 25 oder 26, dadurch gekennzeichnet, daß die Substanz oder die Substanzen ausgewählt sind aus der Gruppe bestehend aus einem Protein, einem Polysaccharid oder einer Nucleinsäure, Mischungen von Proteinen, Mischungen von Polysacchariden oder Mischung von Proteinen oder Polysacchariden gegebenenfalls mit Nucleinsäuren.

28. Mikroteilchen, insbesondere Mikrokapseln, nach einem der Ansprüche 25 oder 27, dadurch gekennzeichnet, daß die Proteine ausgewählt sind aus der Gruppe bestehend aus α-Lactalbumin, β-Lactoglobulin, Caseinen, Ovalbumin, tierischen Albuminen, Blutglobulinen, Hämoglobin, Fibrinogen, Kollagen, Atelokollagen, Gelatine, Keratin, pflanzlichen Albuminen, pflanzlichen Globulinen, Gluteninen, Gliadin; Protein-Extrakten, die von Milch, Soja, Zerealien, Hülsenfrüchten, Algen, Fisch stammen; das Polysaccharid ausgewählt ist aus der Gruppe bestehend aus Agar, Agarose, Agaropektin, Carrageenanen, Alginaten, Pektinen, Amylose, Amylopektin, Amidon, modifizierten Amidonen, Galactomannanen, wie Guar, Johannisbrot, Glucomannan, Konjac, modifizierten Cellulosen, Inulin, Xanthan, Dextran, Curdlan, Gellan, Chitosan, Chondroitinsulfaten, Hyaluronsäure, Dermatansulfat, Heparansulfat, Heparin, Keratansulfat; und die Nucleinsäure ausgewählt ist aus Ribonucleinsäure und Desoxyribonucleinsäure.

29. Mikroteilchen, insbesondere Mikrokapseln, nach einem der Ansprüche 25 bis 28, dadurch gekennzeichnet, daß sich das Polysaccharid spezifisch mit den Kationen oder Polykationen assoziiert, um ein Gel zu bilden.

30. Mikroteilchen, insbesondere Mikrokapseln, nach Anspruch 29, dadurch gekennzeichnet, daß das Polysaccharid ausgewählt ist aus der Gruppe bestehend aus einem Kappa- oder Iota-Carrageenan, einem schwach methylierten Pektin, einem Alginat und Gellan-Gummi.

31. Mikroteilchen oder Mikrokapseln nach einem der Ansprüche 25 bis 30, dadurch gekennzeichnet, daß das veresterte Polysaccharid ein Propylenglykolalginat oder ein schwach methyliertes Pektin ist, das eine in Anwesenheit von Calciumionen gelierbare Lösung bildet.

32. Mikroteilchen oder Mikrokapseln nach einem der Ansprüche 25 bis 31, dadurch gekennzeichnet, daß die polyaminierte oder polyhydrolysierte Substanz ausgewählt ist aus Humanserumalbumin, Gelatine, Proteinen von Lactoserum, Ovalbumin, einer Mischung von Atelokollagen und Chondroitinsulfat oder Carboxymethylcellulose.

33. Mikroteilchen, insbesondere Mikrokapseln, nach einem der Ansprüche 25 bis 32, dadurch gekennzeichnet, daß sie lyophilisiert sind.

34. Zusammensetzung, wie eine kosmetische Zusammensetzung oder eine pharmazeutische Zusammensetzung oder eine alimentäre Zusammensetzung, dadurch gekennzeichnet, daß sie Mikroteilchen, insbesondere Mikrokapseln, wie in einem der Ansprüche 25 bis 33 definiert, oder wie durch das Verfahren nach einem der Ansprüche 1 bis 24 erhalten, umfaßt.
